# EUROPEAN PATENT SPECIFICATION

(11) **EP 2 174 597 B1**
(45) Date of publication and mention of the grant of the patent: **20.07.2011**
(21) Application number: 09172289.2
(22) Date of filing: 06.10.2009
(51) Int. Cl.: A61B 17/00

(54) **Dispenser for adhesive newtonian fluid**
Spender für ein haftende newtonsche Flüssigkeit
Distributeur pour fluide newtonien adhésif

(30) Priority: 10.10.2008 IT PR20080063
(43) Date of publication of application: 14.04.2010
(73) Proprietor: GEM S.r.l., 55049 Viareggio (Lucca) (IT)
(72) Inventor: Branchetti, Lodovico, 55041, CAMAIORE (LUCCA) (IT)
(74) Representative: Gotra, Stefano

(56) References cited:
- EP-A- 0 689 874
- EP-A- 1 745 747
- WO-A-2006/023605
- WO-A-2007/002227
- US-A- 6 059 749
- US-A1- 2002 198 564
- US-A1- 2003 187 408
- US-B1- 6 179 862
- US-B1- 6 413 496
- US-E1- R E38 281

## Description

The present invention relates to a dispenser for adhesive Newtonian fluid.

As is well known, a fluid is defined as Newtonian if its viscosity is constant irrespective of the shear stress applied to it. In particular, the present invention finds application in the medical sector, in the dispensing of biologically compatible adhesive materials or glues. Such adhesives are usually applied on biological surfaces during open surgery or laparoscopic operations.

In a Newtonian fluid (i.e. medical cyanoacrilates monomers), the relationship between shear stress and strain rate is linear (the coefficient of linearity being the "viscosity") and independent from other factors, with the exception of temperature. In a non-Newtoninan fluid, the relationship between shear stress and strain rate is non-linear and can even be time-dependent, thus a constant coefficient of viscosity cannot be defined. For example, fibrin is a non-Newtonian fluid, and more precisely a thixotropic fluid since viscosity decreases with duration of stress.

Current state-of-the-art surgical techniques (for example in general, vascular, thoracic and urological surgery) use adhesives of synthetic origin, e.g. cyanoacrylates. The latter are formed by condensing formaldehyde with cyanoacetic ester, such as n-butyl or 2-octyl. Cyanoacrylates are able to polymerize in contact with weak bases, e.g. water or tissue surfaces. Furthermore, such adhesives are well tolerated and have negligible side effects.

There are known techniques for applying cyanoacrylate adhesives or glues by means of portable "dropper" dispensers. The dispensers currently on the market dispense the glue drop by drop or in the form of a continuous flow through a cannula.

"Spray" dispensers capable of spraying the glue over broader areas compared to "dropper" dispensers are also known. The functioning of "spray" dispensers is based on the chemical suspension of individual adhesive agents in flows of gas separated in such a way as to originate an equal number of jets or sprays that mix together in the air or directly on the tissue surface.

For example, the portable dispenser described in US4359049 comprises two syringes for dispensing two non-Newtonian adhesive agents that are then mixed and dispensed through a needle. The adhesive agents are dispensed by injecting two pressurized gas flows. In this manner, the adhesives delivered can reach a surface located at a distance of approximately 20 cm from the needle and adhere thereto, forming a film. Said dispenser, however, does not present any applicative advantage for Newtonian fluids.

The principal disadvantage of the dispenser described in US4359049 resides in the fact that the mixing of the two adhesive agents takes place outside the syringes, directly in the needle, thus proving ineffective. In fact, the final mixture takes on a nonuniform consistency and consequently provides an insufficient adhesive and cohesive strength for use in surgical operations.

Moreover, the gases employed are those generally present in an operating room, such as CO₂. Such gases manifest strong pressure fluctuations which introduce a significant non-uniformity into the sprays.

Furthermore, another disadvantage of the dispenser described is tied to the excessive rapidity of polymerization of the adhesive compounds on the impacted surfaces. In fact, the polymerization times typically range between about 5 and 20 seconds. Due to this characteristic (which in itself would be advantageous for surgical operations), the dispensing cannula becomes easily clogged and the quality of subsequent sprays is impaired.

Another solution, presented in US5116315, illustrates a "spray" dispenser for two adhesive agents provided with spray orifices that can be directly replaced by the user in the event they become clogged. However, the replacement of orifices entails an interruption in the surgical operation underway and undesired wastes of time. Moreover, the replacement may prove difficult for the user.

The document US6432084 describes another "spray" dispensing method. However, this solution exclusively regards non-Newtonian fluids. In particular, said solution employs a type of tip in which the adhesive agents are mixed prior to their impact with the carrier gases. This prevents the compound from polymerizing and obstructing the cannula before being delivered.

However, this solution, too, presents considerable disadvantages. The principal disadvantage resides in the use of gases present in the operating room as propellant gases for the adhesives. As already explained above, such gases manifest strong pressure fluctuations that introduce a significant non-uniformity into the sprays. Consequently, these dispensers do not allow fine deposits of adhesives to be achieved.

A further solution, disclosed in US5154320, describes the use of cyanoacrylates enclosed in an "aerosol"-type protective glass container.

However, the device described is not capable of delivering fluids at distances compatible with laparoscopic surgery.

Moreover, in this case as well, the outlet orifice is subject to frequent clogging.

Furthermore, since it is not possible to sterilize the external packaging of the dispenser, it cannot be used as a class III medical device.

Another disadvantage of this solution is tied to the impossibility of controlling the quantity of fluid delivered.

Another solution, disclosed in US2002/0198564, consists in a gas powered spraying device suitable for single or two-components mixtures delivery. Nevertheless, said device is not suitable for dispensing cyanoacrylates.

A dispenser for medical cyanoacrylates according to the preamble of claim 1 is disclosed in US 6, 179, 862.

In this context, the technical task at the basis of the present invention is to propose a dispenser for adhesive Newtonian fluid which overcomes the limitations of the above-mentioned prior art.

In particular, it is an object of the present invention to provide a dispenser for adhesive Newtonian fluid according to claim 1, such that the fluid dispensed polymerizes to form a thin, uniform film over the target surface.

Another object of the present invention is to propose a dispenser for adhesive Newtonian fluid such that the fluid dispensing cannula or nozzle is not clogged by the polymerization of the fluid.

A further object of the present invention is to provide a dispenser for adhesive Newtonian fluid in which it is possible to control both the quantity of fluid delivered and the area covered thereby.

A further object of the present invention is to provide a dispenser for adhesive Newtonian fluid having a simplified structure compared to the devices existing on the market.

A further object of the present invention is to provide a dispenser for adhesive Newtonian fluid that is easy to use.

Another object of the present invention is to propose a dispenser for adhesive Newtonian fluid usable in the medical sector, for example in open surgery or laparoscopic operations.

The defined technical task and the specified objects hereof are substantially achieved by a dispenser for adhesive Newtonian fluid comprising the technical characteristics described in one or more of the appended claims.

Further characteristics and advantages of the present invention will become more apparent from the following approximate, and hence non-restrictive, description of a preferred, but not exclusive, embodiment of a dispenser for adhesive Newtonian fluid as illustrated in the appended drawings, in which:

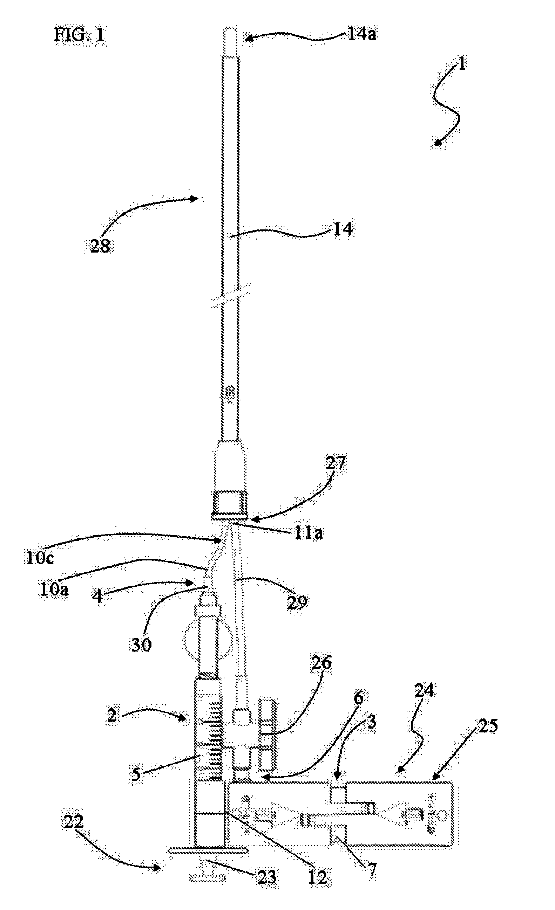

- figure 1 illustrates a lateral view of a dispenser for adhesive Newtonian fluid, according to the present invention;

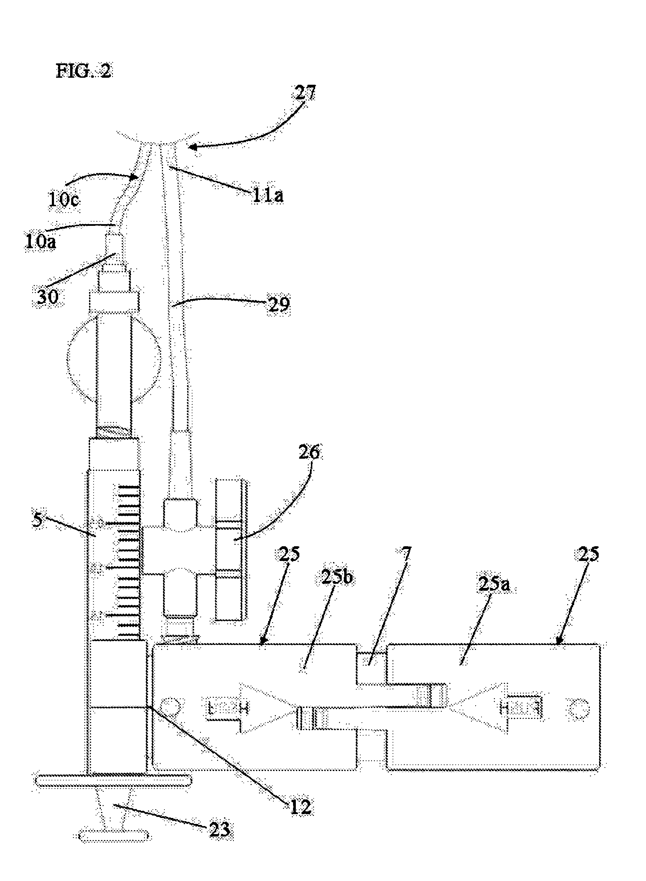

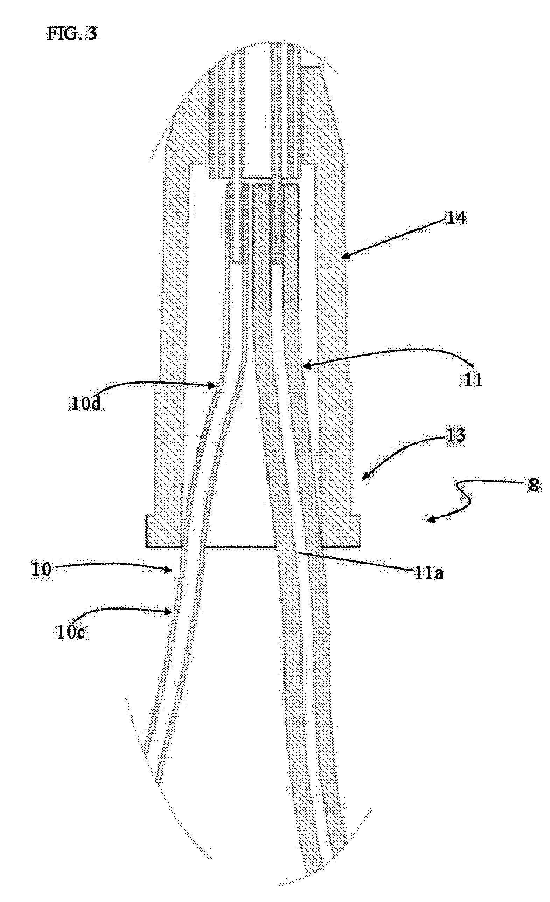

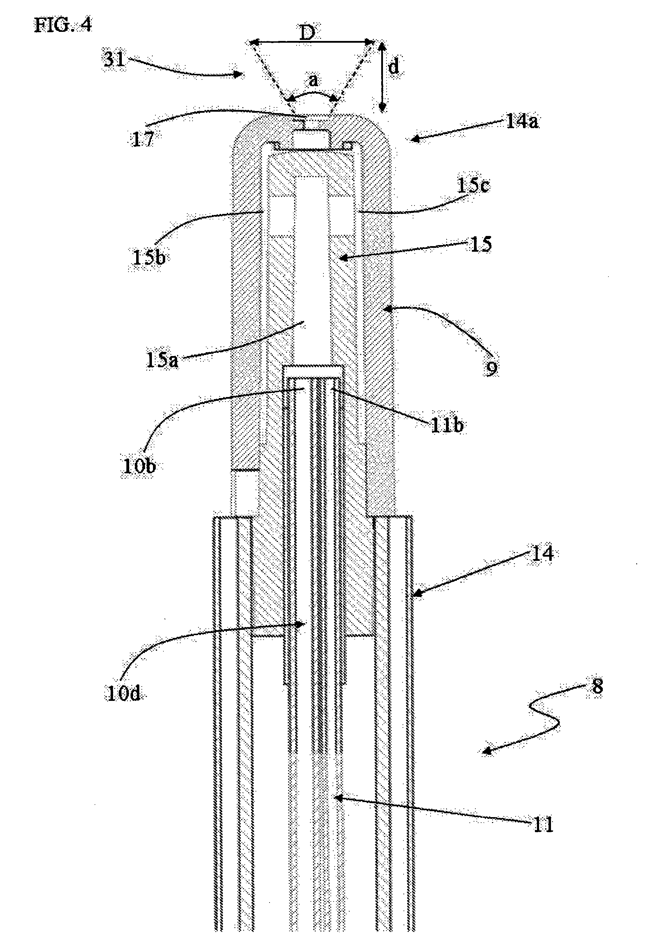

- figures 2, 3 and 4 illustrate a lateral view of different portions of the dispenser of figure 1.

With reference to the figures, a dispenser for adhesive Newtonian fluid has been indicated with the number 1. In particular, the dispenser 1 finds application in the medical sector, preferably in open or laparoscopic surgery.

Preferably, the dispenser 1 is portable. For example, the dispenser 1 is employed as a single-user instrument for sanitary reasons.

In this context, the adhesive Newtonian fluid consists of a biologically compatible adhesive or glue. In particular, the adhesive Newtonian fluid comprises a bioabsorbable component. For example, the adhesive fluid comprises an antibacterial agent. Said fluid can comprise a free radical stabilizer (e.g. hydroquinone, butylated hydroxyanisole or butylated hydroxytoluene) or an anionic stabilizer (e.g. sulfur dioxide or carbonic acid).

Preferably, the adhesive fluid is made up of a medical cyanoacrylate monomer. Alternatively, said fluid comprises a cyanoacrylate and a co-monomer. Cyanoacrylates have good haemostatic and adhesive properties and, once solidified, they create an effective antiseptic barrier against the most common infective and pathogenic agents in surgical interventions.

In particular, the adhesive fluid used is made up of a monomer capable of polymerizing on contact with a moist surface, such as a body tissue. For example, the adhesive fluid has the following structural formula: 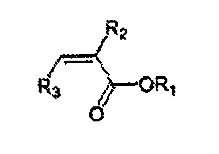 where R₁ is alkyl or alkoxy alkyl or aryl, R₂ is an electron withdrawing group such as CN, CO₂H, Cl, Br or F, while R₃ is alkyl or alkoxy alkyl or aryl.

Preferably, the adhesive fluid comprises a rheological modifier or a plasticizer. Preferably, the adhesive fluid comprises additive agents capable of improving biocompatibility by eliminating or rendering harmless toxic products (e.g. formaldehyde) coming from decomposition.

The dispenser 1 has a first reservoir 2 for the adhesive Newtonian fluid, provided with a first aperture 4 for the egress of the fluid. In the embodiment described herein, the first reservoir 2 is made up of at least one syringe 5 containing the adhesive fluid. The outlet spout of the syringe 5 defines the first aperture 4.

The dispenser 1 is also provided with an inlet 27 for a propellant gas. In particular, the propellant gas is biocompatible and approved for medical use. Preferably, the propellant gas is HFC134/a (1,1,1,2 tetrafluoroethane), that means a non-toxic, biocompatible, non-flammable compound.

Preferably, the dispenser 1 comprises a second reservoir 3 for the propellant gas connected to the inlet 27 of the gas itself. In particular, the second reservoir 3 is provided with a second aperture 6 for the egress of the propellant gas and a duct 29 for guiding the propellant gas so as to connect the second aperture 6 and the inlet 27.

Preferably, said second reservoir 3 is made up of a cylinder 7 containing the gas. Preferably, the cylinder 7 is made of steel or aluminium. In particular, the inner walls of the cylinder 7 are coated with an inert material, such as epoxy resin. The gas present in the cylinder 7 is under pressure, thus the cylinder 7 must be protected from sources of heat.

The dispenser 1 is provided with means 28 for mixing the adhesive fluid coming out from the first aperture 4 and the propellant gas coming from the inlet 27 in order to obtain a mixture deliverable on a surface (e.g. a body tissue).

Innovatively, the mixing means 28 are shaped in such a way that the mixing of the adhesive fluid and the propellant gas takes place in proximity to an open end 14a of a cannula or catheter 14 of the dispenser 1.

Advantageously, the mixture is atomized from the open end 14a so as to reach the surface and be deposited on the latter.

The dispenser 1 is provided with guide means 8 for the adhesive fluid and the propellant gas. Said guide means 8 are connected to the first aperture 4 and to the inlet 27 for the gas so as to receive the adhesive fluid and the propellant gas and guide them separately towards a confluence element 9.

Advantageously, the confluence element 9 is positioned in correspondence to the open end 14a.

In the embodiment described herein, the guide means 8 comprise at least one first duct 10 for the adhesive fluid and at least one second duct 11 for the propellant gas.

In particular, the first duct 10 has an entrance 10a connected to the first aperture 4 and an outlet 10b connected to the confluence element 9. Preferably, the entrance 10a of the first duct 10 is connected to the first aperture 4 by means of a "luer" coupling. The first duct 10 is made up of an initial tract 10c connected to the entrance 10a and of a final tract 10d connected to the outlet 10b. Preferably, the final tract 10d has a smaller diameter than the initial tract 10c.

Preferably, the first duct 10 is provided with a nonreturn valve 30 to prevent the propellant gas from entering the first reservoir 2.

Preferably, the second duct 11 has an entrance 11a connected to the inlet 27 for the propellant gas and an outlet 11b connected to the confluence element 9.

Advantageously, the duct 29 for guiding the gas and the initial tract 10c of the first duct 10 are disposed in such a way as to converge at a funnel 13. The second duct 11 and the final tract 10d of the first duct 10 branch off from said funnel 13. In particular, the second duct 11 and the final tract 10d of the first duct 10 are substantially parallel.

Preferably, the catheter 14 contains at least a portion of the second duct 11 and at least the final tract 10d of the first duct 10. In particular, the catheter 14 is flexible and has a length of around 33 cm and a diameter of around 5 mm. Preferably, the catheter 14 is made up of plastic material approved for medical use, such as polyethylene, polypropylene or PTFE.

Innovatively, the confluence element 9 comprises a diffusing tip 15 provided with a first channel 15a in which the meeting of the adhesive fluid and the propellant gas takes place, and a second channel 15b and a third channel 15c branching off from said first channel 15a. Advantageously, the second channel 15b and the third channel 15c have a smaller diameter than the first channel 15a in such a way as to increase the pressure of the mixture. In particular, the diffusing tip 15 is provided with a nozzle 17 to which the second channel 15b and the third channel 15c are afferent in such a way as to atomize the mixture.

The dispenser 1 is provided with activating means 22 for the adhesive fluid to produce the egress of the fluid from the first aperture 4 to the mixing means 28. Said activating means 22 for the fluid are manual or automatic. For example, the activating means 22 for the fluid comprise a plunger 23 to push the fluid out of the syringe 5 through the first aperture 4.

The dispenser 1 is moreover provided with activating means 24 for the propellant gas to produce the egress of the gas from the second aperture 6 towards the duct 29 for guiding the gas. Said activating means 24 for the gas are manual or automatic. In particular, in the embodiment described herein, the activating means 24 for the gas comprise two complementary half-shells 25 inside which the second reservoir 3 is housed. In the embodiment presented herein, the cylinder 7 is housed between a lower half-shell 25a and an upper half-shell 25b. Preferably, the half-shells 25 are made up of plastic material, e.g. ABS (acrylonitrile-butadienestyrene). Said half-shells 25 are displaceable from a position of rest in which they are spaced apart from each other, to an operative position where they are brought together so as to latch onto each other.

Preferably, on the outside of the upper half-shell 25b there is a housing 12 for the syringe 5.

Preferably, the activating means 24 for the gas comprise a tap 26 operatively active on the guiding duct 29 for the propellant gas. In particular, said tap 26 is displaceable from a closure configuration in which it prevents the propellant gas from continuing towards the inlet 27 for the gas, to a plurality of opening configurations in which it allows the propellant gas to reach the inlet 27 in pre-established quantities.

Alternatively, in the absence of the tap 26, the gas coming out of the second aperture 6 travels continuously toward the inlet 27 for the gas.

The functioning of the dispenser for adhesive Newtonian fluid, according to the present invention, is described below.

The dispenser 1 is gripped with one hand by a user. Preferably, the dispenser 1 is taken hold of by the half-shells 25 in such a way that the cylinder 7 containing the propellant gas is in a lower position than the syringe 5 and the catheter 14 is substantially horizontal. Preferably, the tip 15 is situated at a distance of between 2 cm and 5 cm from the surface to be atomized onto.

With his/her hand engaged, the user brings the two half-shells 25 from the position of rest to the operative position, thereby causing them to latch together. In this way, the gas flows out from the second aperture 6 toward the guiding duct 29.

In the absence of the tap 26, the gas then continues toward the inlet 27 for the gas. In the presence of the tap 26, the user turns the tap 26 in such a way as to bring it from the closure configuration to one of the opening configurations so as to enable a pre-established quantity of gas to reach the inlet 27. Said pre-established quantity of gas thus arrives in the second duct 11.

The user then presses on the plunger 23 of the syringe 5 in such a way as to push the fluid out of the first aperture 4 toward the initial tract 10c of the first duct 10. Said fluid thus arrives at the final tract 10d of the first duct 10.

The gas coming from the second duct 11 and the fluid coming from the final tract 10d of the first duct 10 are mixed inside the diffusing tip 15 and atomized through the nozzle 17.

Depending on the pressure exerted on the plunger 23, the opening configuration of the tap 26 and the distance d of the nozzle 17 from the delivery surface, the atomization parameters will vary. In particular, the delivered fluid defines an atomization profile 31 having substantially a truncated cone shape. The opening of said atomization profile 31 is quantifiable by means of an opening angle a or an atomization diameter D. The number of molecules of delivered fluid present within the atomization profile 31 defines a concentration Q of atomization. For example, by placing the nozzle 17 at a distance d from the surface equal to around 5 cm, in optimal conditions an atomization diameter D equal to around 6 cm and an opening angle a of around 60° will be obtained.

Preferably, once the egress of the fluid from the first aperture 4 ceases (which occurs almost instantaneously when the pressure on the plunger 23 is interrupted), it is necessary to wait a few seconds before bringing the tap 26 back into the closure configuration. This allows the nozzle 17 to be cleaned by the passage of the propellant gas.

The characteristics of the dispenser for adhesive Newtonian fluid, according to the present invention, emerge clearly from the description provided, as do the advantages thereof.

In particular, thanks to the configuration of the diffusing tip, the propellant gas and the adhesive fluid are uniformly mixed and premature polymerization in the nozzle is prevented.

Furthermore, the three channels of the diffusing tip enable the fluid to be atomized, i.e. to be delivered homogeneously across space and thus form a thin film on the surface concerned. Moreover, the second and the third channel of the diffusing tip, having a smaller diameter than the first channel, serve to reduce the contamination of the atomized fluid by airborne dusts.

Furthermore, thanks to the fact that the gas continues to come out for a few seconds after the egress of fluid from the first reservoir ceases, cleaning of the diffusing tip is assured, thus preventing the occurrence of undesired clogging.

Moreover, since the delivery of the fluid takes place at the open end of the catheter, the times of response to the dispensing command (i.e. from the instant at which the plunger is pressed) are practically immediate, allowing the user full control of the quantity of fluid to be delivered.

Furthermore, thanks to the adjustment of the tap and the possibility of varying the distance d between the nozzle and the delivery surface, it is possible to always keep both the quantity of fluid to be delivered (i.e. the concentration Q of atomization) and the area covered (i.e. the opening angle a and the atomization diameter D) under control.

Moreover, the dispenser presents a very simple structure, since the adhesive fluid is contained in the syringe and the propellant gas is in the cylinder. The housing of the syringe above the cylinder also contributes to rendering use of the dispenser easy and ergonomic.

Furthermore, the dispenser is easy to handle thanks to the presence of the two half-shells containing the cylinder and the use of a simple plunger to activate the egress of the fluid. The presence of the catheter also contributes to rendering the dispenser easier to handle, in addition to performing an important protective function for the guiding means for the fluid and for the gas.

Finally, since the delivery of the fluid takes place by atomization, the proposed dispenser can be used in open surgery and laparoscopic operations.

## Claims

1. Dispenser (1) for adhesive medical cyanoacrylate comprising:
a first reservoir (2) containing adhesive medical cyanoacrylate, provided with a first aperture (4) for the egress of said adhesive medical cyanoacrylate;
an inlet (27) for a propellant gas;
means (28) for mixing the adhesive medical cyanoacrylate coming out from the first aperture (4) and the propellant gas coming from said inlet (27) in order to obtain a mixture deliverable on a surface;
guide means (8) for the adhesive medical cyanoacrylate and the propellant gas connected to the first aperture (4) and to the inlet (27) for the gas so as to receive the adhesive medical cyanoacrylate and the propellant gas,
wherein said mixing means (28) are shaped in such a way that the mixing of the adhesive medical cyanoacrylate and the propellant gas takes place in proximity to an open end (14a) of the cannula or catheter (14) of the dispenser (1), said mixture being atomized from said open end (14a) onto the surface,
**characterised in that** said guide means guide the adhesive medical cyanocrylate and the propellant gas separately towards a confluence element (9) positioned in correspondence to said open end (14a) of the cannula or catheter (14) of the dispenser (1), and
**in that** said confluence element (9) comprises a diffusing tip (15) provided with a first channel (15a) in which the meeting of the adhesive medical cyanoacrylate and the propellant gas takes place, and a second channel (15b) and a third channel (15c) branching off from said first channel (15a) and having smaller diameter than said first channel (15a) in such a way as to increase the pressure of the mixture.

2. Dispenser (1) according to claim 1, **characterised in that** it comprises means (22) of activating the adhesive fluid to produce the egress of said adhesive fluid from the first aperture (4) to the mixing means (28).

3. Dispenser (1) according to claim 2, wherein the first reservoir (2) is made up of at least one syringe (5) containing the adhesive fluid, and the activating means (22) for the adhesive fluid comprise a plunger (23) to push the adhesive fluid out of said at least one syringe (5), the outlet spout of the syringe (5) defining the first aperture (4).

4. Dispenser (1) according to any of the preceding claims, **characterised in that** it comprises a second reservoir (3) for the propellant gas, connected to said inlet (27) for the propellant gas.

5. Dispenser (1) according to claim 4, **characterised in that** it comprises means (24) of activating the propellant gas to produce the egress of the gas from a second aperture (6) of the second reservoir (3) towards a duct (29) for guiding said propellant gas.

6. Dispenser (1) according to claim 5, wherein the activating means (24) for the gas comprise a tap (26) operatively active on the guiding duct (29) and displaceable from a closure configuration in which it prevents the propellant gas from continuing towards the inlet (27) for the gas, to a plurality of opening configurations in which it allows the propellant gas to reach said inlet (27) in pre-established quantities.

7. Dispenser (1) according to claim 5 or 6, wherein the activating means (24) for the gas comprise two complementary half-shells (25) inside which the second reservoir (3) is housed, said half-shells (25) being displaceable from a position of rest in which they are spaced apart from each other, to an operative position where they are brought together so as to latch onto each other.

8. Dispenser (1) according to claim 1, wherein the guide means (8) comprise at least one first duct (10) for the adhesive fluid and at least one second duct (11) for the propellant gas, said at least one first duct (10) having an entrance (10a) connected to the first aperture (4) and an outlet (10b) connected to the confluence element (9) and said at least one second duct (11) having an entrance (11a) connected to the inlet (27) for the propellant gas and an outlet (11b) connected to the confluence element (9).

9. Dispenser (1) according to claim 8, wherein said first duct (10) is made up of an initial tract (10c) connected to the entrance (10a) and of a final tract (10d) connected to the outlet (10b), the final tract (10d) having a smaller diameter than the initial tract (10c).

10. Dispenser (1) according to claims 8 or 9, wherein the first duct (10) is provided with a nonreturn valve (30) for preventing the propellant gas from entering the first reservoir (2).

11. Dispenser (1) according to claim 1, wherein the diffusing tip (15) is provided with a nozzle (17) to which the second channel (15b) and the third channel (15c) are afferent in such a way as to atomize the mixture.

12. Dispenser (1) according to any of the preceding claims, wherein the propellant gas is biocompatible and approved for medical use.

13. Dispenser (1) according to any of the preceding claims, wherein the propellant gas is HFC134/a (1,1,1,2 tetrafluoroethane).

14. Dispenser (1) according to any of the preceding claims, wherein the delivered fluid defines an atomization profile (31) having substantially a truncated cone shape, an atomization diameter (D) equal to around 6 cm and an opening angle (a) of around 60° are obtained in condition by placing said nozzle (17) at a distance (d) equal to around 5 cm from said surface.

15. Dispenser (1) according to any of the preceding claims, wherein said dispenser is portable.

## Patentansprüche

1. Spender (1) für haftendes medizinisches Cyanacrylat, umfassend
einen ersten Behälter (2), der haftendes medizinisches Cyanacrylat enthält und mit einer ersten Öffnung (4) für den Austritt des genannten haftenden medizinischen Cyanacrylats versehen ist;
einen Einlass (27) für ein Treibgas;
Mittel (28) zum Mischen des haftenden medizinischen Cyanacrylats, das aus der ersten Öffnung (4) heraustritt, und des Treibgases, das aus dem genannten Einlass (27) kommt, um eine Mischung zu erhalten, die auf eine Oberfläche geleitet werden kann;
Führungsmittel (8) für das haftende medizinische Cyanacrylat und das Treibgas, die mit der ersten Öffnung (4) und dem Einlass (27) für das Gas verbunden sind, sodass das haftende medizinische Cyanacrylat und das Treibgas empfangen werden, wobei
die genannten Mittel zum Mischen (28) so geformt sind, dass das Mischen des haftenden medizinischen Cyanacrylats und des Treibgases in der Nähe eines offenen Endes (14a) der Kanüle oder des Katheters (14) des Spenders (1) stattfindet, wobei die Mischung vom genannten offenen Ende (14a) auf der Oberfläche zerstäubt wird,
**dadurch gekennzeichnet, dass** die genannten Führungsmittel das haftende medizinische Cyanacrylat und das Treibgas separat zu einem Zusammenflusselement (9) führen, das am genannten offenen Ende (14a) der Kanüle oder des Katheters (14) des Spenders positioniert ist und **dadurch**, dass
das genannte Zusammenflusselement (9) eine Verteilerspitze (15) umfasst, die mit einem ersten Kanal (15a) ausgestattet ist, in dem das haftende medizinische Cyanacrylat und das Treibgas aufeinander treffen, sowie einem zweiten Kanal (15b) und einem dritten Kanal (15c), die vom genannten ersten Kanal (15a) abzweigen und einen geringeren Durchmesser als der genannte erste Kanal (15a) aufweisen, sodass der Druck der Mischung erhöht werden kann.

2. Spender (1) nach Anspruch 1, **dadurch gekennzeichnet, dass** er Mittel (22) zum Aktivieren der haftenden Flüssigkeit umfasst, um den Austritt der genannten haftenden Flüssigkeit von der ersten Öffnung (4) zu den Mitteln zum Mischen (28) zu bewirken.

3. Spender (1) nach Anspruch 2, wobei der erste Behälter (2) aus mindestens einer Spritze (5) besteht, die die haftende Flüssigkeit enthält, und die Mittel zum Aktivieren (22) für die haftende Flüssigkeit einen Kolben (23) umfassen, um die haftende Flüssigkeit aus der mindestens einen Spritze (5) herauszudrücken, wobei die Auslasstülle der Spritze (5) die erste Öffnung (4) definiert.

4. Spender (1) nach einem der vorhergehenden Ansprüche, **dadurch gekennzeichnet, dass** er einen zweiten Behälter (3) für das Treibgas umfasst, der mit dem genannten Einlass (27) für das Treibgas verbunden ist.

5. Spender (1) nach Anspruch 4, **dadurch gekennzeichnet, dass** er Mittel (24) zum Aktivieren des Treibgases umfasst, um den Austritt des Gases aus einer zweiten Öffnung (6) des zweiten Behälters (3) zu einer Leitung (29) zum Führen des genannten Treibgases zu bewirken.

6. Spender (1) nach Anspruch 5, wobei die Mittel zum Aktivieren (24) für das Treibgas einen Zapfen (26) umfassen, der operativ auf der Führungsleitung (29) aktiv ist und von einer Schließkonfiguration, in der er verhindert, dass das Treibgas weiter in Richtung des Einlasses (27) für das Gas, geleitet wird, in eine Vielzahl von Öffnungskonfigurationen verstellt werden kann, in denen er dem Treibgas ermöglicht, den genannten Einlass (27) in vorgegebenen Mengen zu erreichen.

7. Spender (1) nach Anspruch 5 oder 6, wobei die Mittel zum Aktivieren (24) für das Gas zwei sich ergänzende Hüllenhälften (25) umfassen, in denen der zweite Behälter (3) untergebracht ist, wobei die genannten Hüllenhälften (25) von einer Ruhestellung, in der sie voneinander entfernt sind, in eine Arbeitsstellung verstellt werden können, in der sie aneinander angenähert werden, sodass sie einrasten.

8. Spender (1) nach Anspruch 1, wobei die Führungsmittel (8) mindestens eine erste Leitung (10) für die haftende Flüssigkeit und mindestens eine zweite Leitung (11) für das Treibgas umfassen, die genannte mindestens eine erste Leitung (10) einen Eingang (10a), der mit der ersten Öffnung (4) verbunden ist, und einen Auslass (10b), der mit dem Zusammenflusselement (9) verbunden ist, aufweist, und die genannte mindestens eine zweite Leitung (11) einen Eingang (11a), der mit dem Einlass (27) für das Treibgas verbunden ist, und einen Auslass (11b), der mit dem Zusammenflusselement (9) verbunden ist, aufweist.

9. Spender (1) nach Anspruch 8, wobei die genannte erste Leitung (10) aus einem anfänglichen Teilstück (10c) besteht, das mit dem Eingang (10a) verbunden ist, und einem abschließenden Teilstück (10d), das mit dem Auslass (10b) verbunden ist, wobei das abschließende Teilstück (10d) einen geringeren Durchmesser als das anfängliche Teilstück (10c) besitzt.

10. Spender (1) nach Anspruch 8 oder 9, wobei die erste Leitung (10) mit einem Rückschlagventil (30) ausgestattet ist, damit das Treibgas nicht in den ersten Behälter (2) eingeleitet wird.

11. Spender (1) nach Anspruch 1, wobei die Verteilerspitze (15) mit einer Düse (17) ausgestattet ist, zu dem der zweite Kanal (15b) und der dritte Kanal (15c) so hinführen, dass die Mischung zerstäubt wird.

12. Spender (1) nach einem der vorhergehenden Ansprüche, wobei das Treibgas biokompatibel und für den medizinischen Gebrauch zugelassen ist.

13. Spender (1) nach einem der vorhergehenden Ansprüche, wobei das Treibgas HFC134/a (1,1,1,2 Tetrafluorethan) ist.

14. Spender (1) nach einem der vorhergehenden Ansprüche, wobei die zugeführte Flüssigkeit ein Zerstäubungsprofil (31) definiert, das im Wesentlichen eine stumpfkegelförmige Form aufweist, wobei ein Zerstäubungsdurchmesser (D) gleich zirka 6 cm und ein Öffnungswinkel (a) von zirka 60° erzielt werden, wenn die genannte Düse (17) auf einer Entfernung (d) gleich zirka 5 cm von der genannten Oberfläche platziert wird.

15. Spender (1) nach einem der vorhergehenden Ansprüche, wobei der genannte Spender tragbar ist.

## Revendications

1. Distributeur (1) pour cyanoacrylate adhésif à usage médical comprenant:
un premier réservoir (2) contenant du cyanoacrylate adhésif à usage médical, muni d'une première ouverture (4) pour la sortie dudit cyanoacrylate adhésif à usage médical;
une entrée (27) de gaz propulseur;
moyens (28) pour mélanger le cyanoacrylate adhésif à usage médical sortant de la première ouverture (4) et le gaz propulseur sortant de ladite entrée (27) afin d'obtenir un mélange transférable sur une surface;
moyens de guidage (8) pour le cyanoacrylate adhésif à usage médical et le gaz propulseur reliés à la première ouverture (4) et à l'entrée (27) de gaz de telle sorte à recevoir le cyanoacrylate adhésif à usage médical et le gaz propulseur, dans lequel
lesdits moyens (28) pour mélanger sont formés de telle sorte que le mélange du cyanoacrylate adhésif à usage médical et du gaz propulseur se réalise à proximité d'une extrémité ouverte (14a) d'une canule ou d'un cathéter (14) du distributeur (1), ledit mélange étant atomisé à partir de ladite extrémité ouverte (14a) sur la surface,
**caractérisé en ce que** lesdits moyens de guidage guident le cyanoacrylate adhésif à usage médical et le gaz propulseur séparément vers un élément de convergence (9) positionné en correspondance de ladite extrémité ouverte (14a) de la canule ou du cathéter(14) du distributeur et
**en ce que** ledit élément de convergence (9) comprend un embout diffuseur (15) muni d'un premier canal (15a) dans lequel la rencontre du cyanoacrylate adhésif à usage médical et du gaz propulseur se réalise, et un second canal (15b) et un troisième canal (15c) branchés à partir dudit premier canal (15a) et ayant un plus petit diamètre que ledit premier canal (15a) de telle sorte à augmenter la pression du mélange.

2. Distributeur (1) selon la revendication 1, **caractérisé en ce qu'**il comprend les moyens (22) d'activation du fluide adhésif pour provoquer la sortie dudit fluide adhésif de la première ouverture (4) vers les moyens pour mélanger (28).

3. Distributeur (1) selon la revendication 2, dans lequel le premier réservoir (2) est composé d'au moins une seringue (5) contenant le fluide adhésif, et les moyens d'activation (22) du fluide adhésif comprennent un piston plongeur (23) pour pousser le fluide adhésif hors de ladite au moins une seringue (5), l'orifice de la sortie de la seringue (5) définissant la première ouverture (4).

4. Distributeur (1) selon l'une quelconque des revendications précédentes, **caractérisé en ce qu'**il comprend un second réservoir (3) pour le gaz propulseur, relié à ladite sortie (27) du gaz propulseur.

5. Distributeur (1) selon la revendication 4, **caractérisé en ce qu'**il comprend les moyens (24) d'activation du gaz propulseur pour provoquer la sortie du gaz à partir de la seconde ouverture (6) du second réservoir (3) vers une conduite (29) pour guider ledit gaz propulseur.

6. Distributeur (1) selon la revendication 5, dans lequel les moyens d'activation (24) du gaz comprennent un robinet (26) opérationnellement actif sur la conduite de guidage (29) et déplaçable d'une configuration de fermeture dans laquelle il empêche le gaz propulseur de circuler vers l'entrée (27) de gaz, vers une pluralité de configurations d'ouverture dans lesquelles il permet au gaz propulseur d'atteindre ladite entrée (27) dans des quantités prédéterminées.

7. Distributeur (1) selon les revendications 5 ou 6, dans lequel les moyens d'activation (24) du gaz comprennent deux demi-coques (25) complémentaires à l'intérieur desquelles le second réservoir (3) est logé, lesdites demi-coques (25) étant déplaçables d'une position de repos, dans laquelle elles sont espacées l'une de l'autre, à une position opérationnelle où elles sont assemblées de telle sorte à être attaché l'une à l'autre.

8. Distributeur (1) selon la revendication 1, dans lequel les moyens de guidage (8) comprennent au moins une première conduite (10) pour le fluide adhésif et au moins une seconde conduite (11) pour le gaz propulseur, ladite au moins première conduite (10) ayant un accès (10a) relié à la première ouverture (4) et une sortie (10b) reliée à l'élément de convergence (9) et ladite au moins seconde conduite (11) ayant une entrée (11a) reliée à l'entrée (27) de gaz propulseur et une sortie (11b) reliée à l'élément de convergence (9).

9. Distributeur (1) selon la revendication 8, dans lequel ladite première conduite (10) est composée d'une partie initiale (10c) reliée à l'accès (10a) et d'une partie finale (10d) reliée à la sortie (10b), la partie finale (10d) ayant un plus petit diamètre que la partie initiale (10c).

10. Distributeur (1) selon les revendications 8 ou 9, dans lequel la première conduite (10) est composée d'un clapet de non-retour (30) pour empêcher le gaz propulseur d'entrer dans le premier réservoir (2).

11. Distributeur (1) selon la revendication 1, dans lequel l'embout diffuseur (15) est composé d'une buse (17) à laquelle le deuxième canal (15b) et le troisième canal (15c) sont afférents de telle sorte à atomiser le mélange.

12. Distributeur (1) selon l'une quelconque des revendications précédentes, dans lequel le gaz propulseur est biocompatible et autorisé à l'usage médical.

13. Distributeur (1) selon l'une quelconque des revendications précédentes, dans lequel le gaz propulseur est du HFC-134a (tétrafluoroéthane).

14. Distributeur (1) selon l'une quelconque des revendications précédentes, dans lequel le fluide délivré définit un profil d'atomisation (31) ayant substantiellement une forme de cône tronqué, et dans lequel un diamètre d'atomisation (D) égal à environ 6 cm et un angle d'ouverture (a) d'environ 60° sont obtenus dans ces conditions en plaçant ladite buse (17) à une distance (d) égale à environ 5 cm de ladite surface.

15. Distributeur (1) selon l'une quelconque des revendications précédentes, dans lequel ledit distributeur est portable.
